# EUROPEAN PATENT SPECIFICATION

(11) **EP 2 079 524 B1**
(45) Date of publication and mention of the grant of the patent: **18.05.2011**
(21) Application number: 07826806.7
(22) Date of filing: 19.10.2007
(51) Int. Cl.: A61N 7/00, A61B 17/22, B06B 1/06

(54) **SYMMETRIC AND PREFERENTIALLY STEERED RANDOM ARRAYS FOR ULTRASOUND THERAPY**
SYMMETRISCHE UND PRÄFERENZIELL GESTEUERTE RANDOM-ARRAYS FÜR ULTRASCHALLTHERAPIE
ENSEMBLES ALÉATOIRES SYMÉTRIQUES ET ORIENTÉS DE MANIÈRE PRÉFÉRENTIELLE POUR UNE THÉRAPIE ULTRASONORE

(30) Priority: 23.10.2006 US 862525 P
(43) Date of publication of application: 22.07.2009
(73) Proprietor: Koninklijke Philips Electronics N.V., 5621 BA Eindhoven (NL)
(72) Inventor: RAJU, Balasundara, Briarcliff Manor, New York 10510-8001 (US); HALL, Christopher S., Briarcliff Manor, New York 10510-8001 (US)
(74) Representative: Schouten, Marcus Maria
(86) International application number: PCT/IB2007/054271
(87) International publication number: WO 2008/050278

(56) References cited:
- WO-A-97/17018
- US-B1- 6 500 141

## Description

The technical field of this invention is ultrasound phased array transducers for therapeutic applications such as tissue ablation, drug delivery, gene delivery, hyperthermia and clot dissolution.

Ultrasound has many therapeutic applications such as tissue ablation, drug delivery, hyperthermia, and clot dissolution. Single element therapy transducers have the limitation of a fixed focal length and the need for mechanical translation to treat different regions of the body. In order to steer the therapy beam to different locations in the body, electronically phased array transducers are generally employed. It is well known in the art that electronically steered arrays can lead to the production of grating lobes that result in undesired energy deposition when the element spacing is not fine enough.

Random and quasi-random arrays have previously been shown for ultrasound therapy applications. E. Hutchinson et al., Aperiodic ultrasound phased array, PCT patent application serial number WO 97/17018; J.F. Hand et al., Arrays of quasi-randomly distributed ultrasound transducers, PCT patent application serial number WO 00/49598. In such arrays the distribution of the elements is randomized. Quasi-random arrays utilize randomized elements whose location, shape, and the size are within user-defined certain bounds. Such a randomization causes a desired loss of regularity, having the effect of reducing the deposition of energy in the grating lobes.

Design of an optimal random array requires solving a large number of unknown parameters. Location, shape, and the size of the elements that make up the array are in general random values, subject to certain bounds. An optimal array is chosen based on minimization of a cost function, which is commonly taken to be the worst (highest) ratio of intensities of each of the grating lobe to the main lobe when the beam is steered to focus, at each of many locations.

Failure of optimization methods based on gradient searches is related to presence of a large number of local minima. Adaptive search algorithms such as genetic algorithms are difficult to implement in a straightforward manner due to the highly constrained nature of the problem (elements must not overlap). In trying to pose the adaptive search problem in a constraint-free manner, one might restrict the solution space to a class of problems where the solution set can be selected in a constraint-free manner. Thus, in practice, a restricted exhaustive search is done in designing the random array. E.B. Hutchinson, et al., Design & optimization of an aperiodic ultrasound phased array for intracavity prostate thermal therapies, 23(5) MED. PHYS. 767-76 (1996). One method used in the prior art is that element sizes are restricted to two or three values of sizes. *Id.* Because of the restricted search, a sub-optimal set is often selected.

Accordingly, a featured embodiment of the invention herein is a phased ultrasound array for an ultrasound generating device, the phased ultrasound array including a symmetrically constrained plurality of randomly selected ultrasound elements partitioned into N portions with 1/N symmetry, in which N represents an integer greater than or equal to two, wherein the randomly selected ultrasound elements of each portion of the array are arranged symmetrically about one of (i) a point or (ii) a dividing line in the array with respect to element sizes, shapes, and element locations, wherein the randomly selected ultrasound elements of a portion include at least one element of the plurality of elements which has a size and/or shape that is different from the remainder of the plurality of elements in the respective portion and/or at least one inter-element spacing that is different from a remainder of inter-element spacings in the respective portion.. In a related embodiment, sizes and/or shapes of the ultrasound elements are randomly selected.

Document US 6,500,141 B 1 discloses an apparatus for treating body tissue, in particular superficial soft tissue, with ultrasound, comprises an ultrasonic generation unit and an applicator, by means of which the ultrasound can be irradiated from an applicator surface facing the body surface from outside through the body surface into the body tissue. A suction apparatus for sucking in the body surface against the applicator surface is provided. This document further discloses a symmetrical arrangement of five individual element ultrasound transducers, operating independently.
In another related embodiment, locations of the ultrasound elements are randomly selected. In another related embodiment, a plurality of the distances between the locations of centers of mass of adjacent ultrasound elements is aperiodic. In another related embodiment, the ultrasound elements of the array are arranged rotationally symmetrically.

Also provided herein is a device for generating ultrasound including any of the phased ultrasound arrays according to any of the above embodiments.

Another featured embodiment of the invention herein is a preferentially steered phased ultrasound array having a plurality of ultrasound elements, such that a majority of larger elements of the ultrasound array are arranged on a first portion of the array and a majority of smaller ultrasound elements are arranged on a second portion of the array, and such that the first portion is closer to a preferred direction of steering than the second portion, the array resulting in greater focusing gain and reduced grating lobes compared to a conventional array having ultrasound elements arranged without regard to ultrasound element size and preferred direction of steering. In a related embodiment, at least one element of the plurality of elements has a size and/or shape that is different from the size and/or shape of the remainder of the plurality of elements, and/or at least one inter-element spacing is different from the spacings of the remainder of the elements. In another related embodiment, the sizes and/or shapes of the ultrasound elements are randomly selected prior to arranging the elements. In another embodiment, the locations of a plurality of ultrasound elements within at least one portion are randomly selected. In another related embodiment, a plurality of ultrasound elements within each portion are randomly arranged.

In another related embodiment, the sizes of the ultrasound elements are randomly selected from two distributions of element sizes: a first distribution of ultrasound element sizes, and a second distribution of ultrasound elements sizes, such that mean size in the first distribution is greater than in the second distribution. In a further related embodiment, the elements of the first portion are randomly selected from the first distribution and the elements of the second portion are randomly selected from the second distribution. In another related embodiment, the first portion and the second portion each constitute about half of the area of the array. In another related embodiment, the first portion and the second portion each constitute half of the area of the array.

In another related embodiment, a plurality of distances between the locations of centers of mass of adjacent ultrasound elements is aperiodic. In yet another related embodiment, the locations of the elements are chosen in a random manner. In a related embodiment, the array in any of the above embodiments is one-dimensional, two-dimensional or three-dimensional. In another related embodiment, the ultrasound elements in any of the above further comprise a material selected from the group consisting of: lead zirconate titanate (PZT), polymer piezo-electric material, and piezo-composite material. In another related embodiment, the ultrasound elements are arranged on a curved surface. For example, the curved surface is a spherical shell.

Another featured embodiment of the invention herein is a method of making a phased ultrasound array, the method including: selecting randomly sized ultrasound elements and arranging the ultrasound elements symmetrically on the array.

Another featured embodiment of the invention herein is a method of making a phased ultrasound array, the method including: selecting randomly sized ultrasound elements and arranging the ultrasound elements wherein the larger ultrasound elements are arranged on a first portion of the array and smaller ultrasound elements are arranged on a second portion of the array, such that the first portion is closer to a preferred direction of steering than the second portion. In a related embodiment, the method further includes optimizing the arrangement of ultrasound elements for steering in an additional direction, wherein optimizing is maintaining focusing gain and minimizing grating lobes.

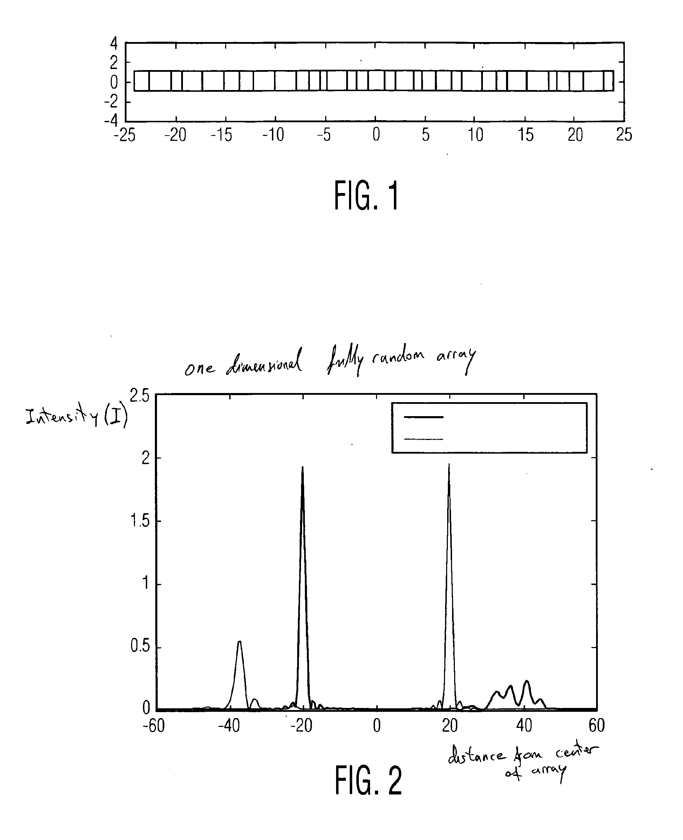

Figure 1 shows an example of a one-dimensional fully random array having a total of 32 elements.
Figure 2 shows field intensity (beam pattern) in arbitrary units as a function of distance on the abscissa from the center of the array (x=0) for a one-dimensional fully random array focused at a plane 80 mm from the aperture. The observed array performance (field intensity) is generally different depending on whether the beam is steered to the right or left. In this example, the array performs more poorly when focused at a point 20 mm off center compared to 20 mm, for example, to the left, from the center.

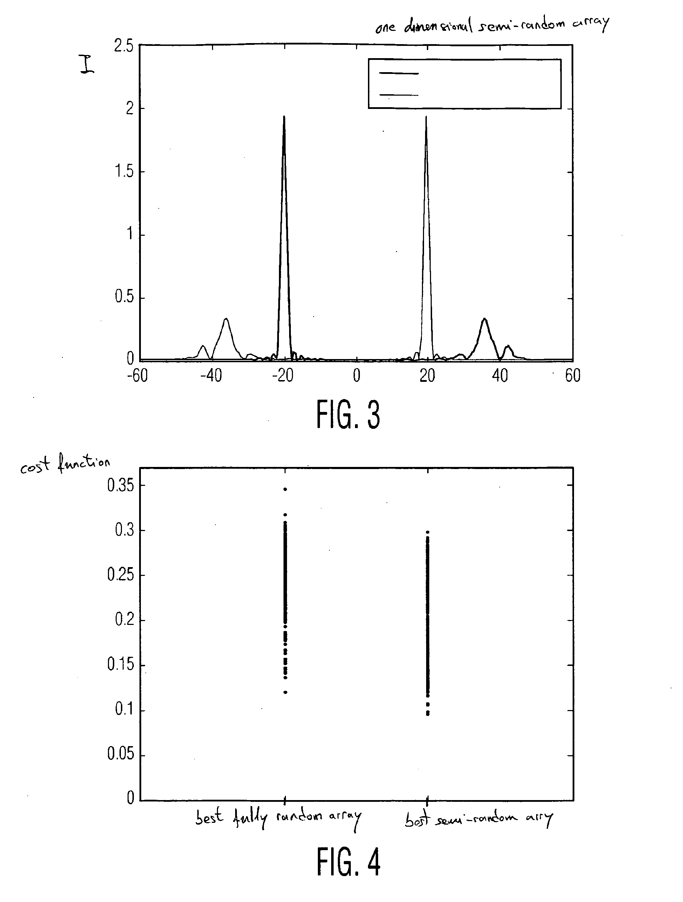

Figure 3 shows the field intensity from a symmetric (semi-random), one-dimensional array of aperture 48 mm and 32 elements. The field distribution is symmetric in that the same level of grating lobe is observed when steered either to the right side or to the left side.
Figure 4 shows a comparison of grating lobe intensity (height) as a fraction of main lobe intensity (height) for fully random arrays and symmetric (semi-) random, one-dimensional arrays for 250 trials. Each dot represents the cost function for one particular trial. Smaller values indicate better performance. The data indicate that the best semi-random array yielded a better (i.e. lower) cost function than the best fully random array.

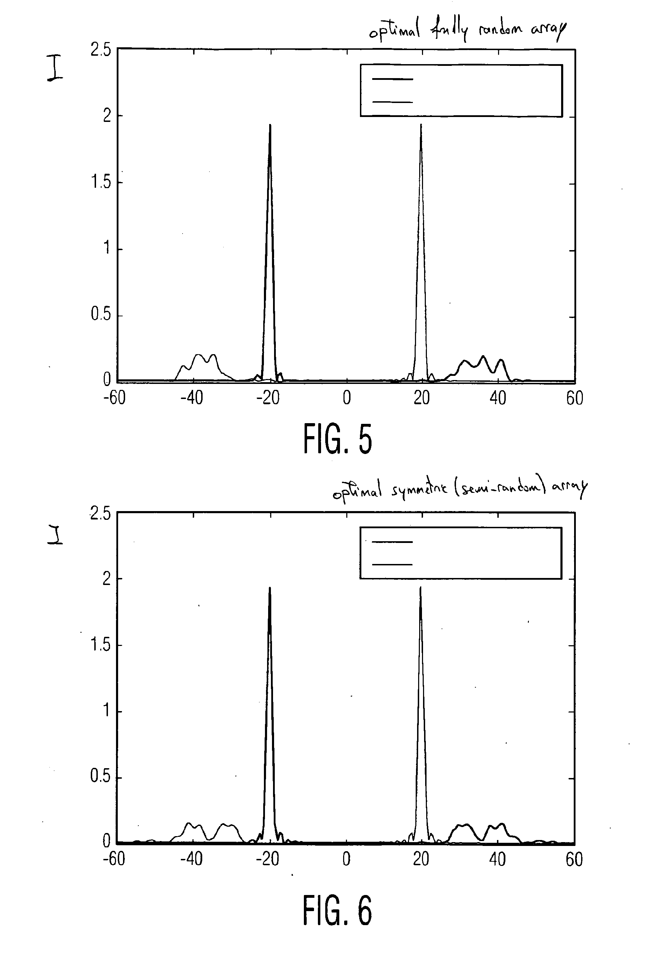

Figure 5 shows the field intensity from an optimal fully random array based on data obtained from 10,000 trials.
Figure 6 shows the field intensity from the optimal symmetric (semi-random) one-dimensional array based on data obtained from 10,000 trials.

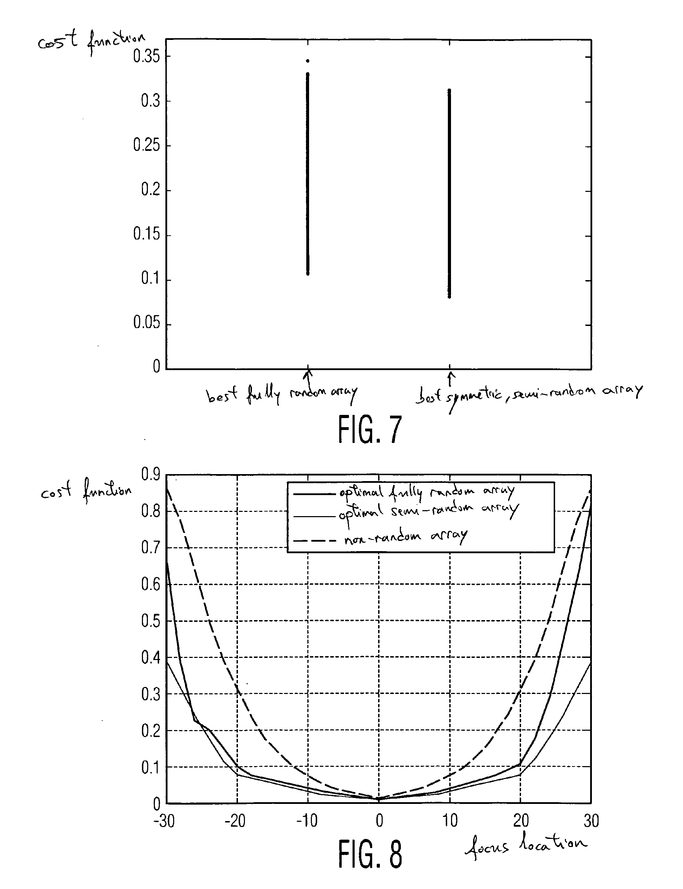

Figure 7 shows a comparison of fully random and symmetric (semi-random), one-dimensional arrays for all the 10,000 trials. Each dot represents the cost function for one particular trial. Smaller value indicates better performance. The data show that the best symmetric (semi-random) array yielded a better cost function than the best fully random array. Even after 10,000 trials, the data obtained using a symmetric (semi-random) design yielded better performance than that with the fully random design.
Figure 8 shows a cost function for optimal fully random and symmetric (semi-random) arrays as a function of the focus location. At greater steering distances from the axis, larger grating lobes occurred. The optimal symmetric (semi-random) array shows better performance than the optimal fully random array (after 10,000 trials). For comparison, the cost function for the non-random array is also shown, which was observed to exhibit a worse (higher) cost function than either the fully random array or the symmetric (semi-random) array.

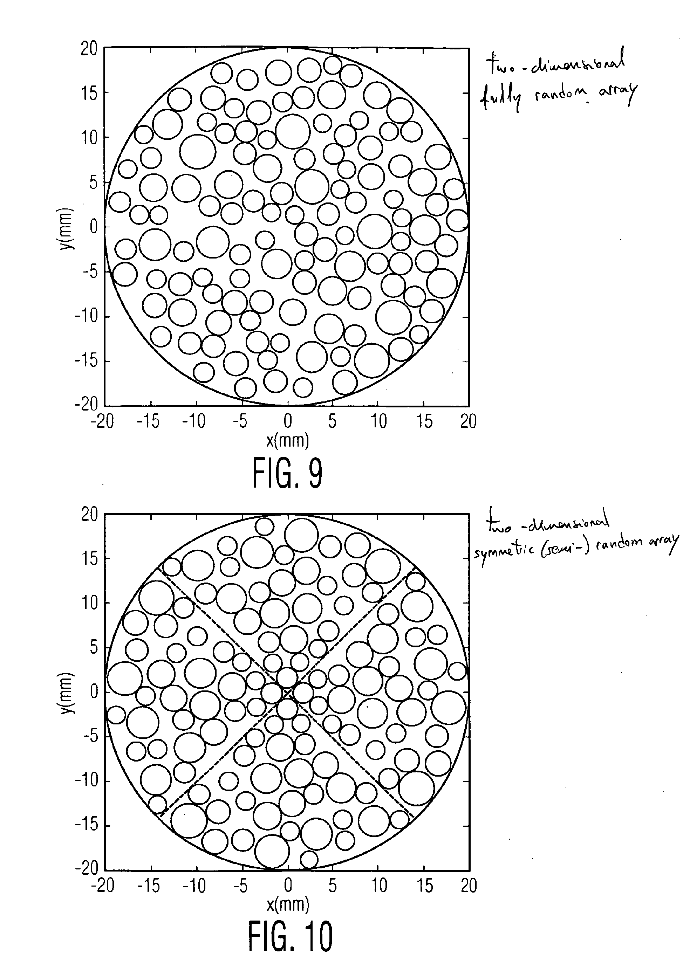

Figure 9 shows an example of a two-dimensional fully random array in a circular aperture.
Figure 10 shows an example of a symmetric two-dimensional random array in a circular aperture. A 90 degree sector of the array is populated with randomly sized and located elements. This sector is then rotated and reproduced at 90, 180 and 270 degrees to generate a rotationally (radially) symmetrical array.

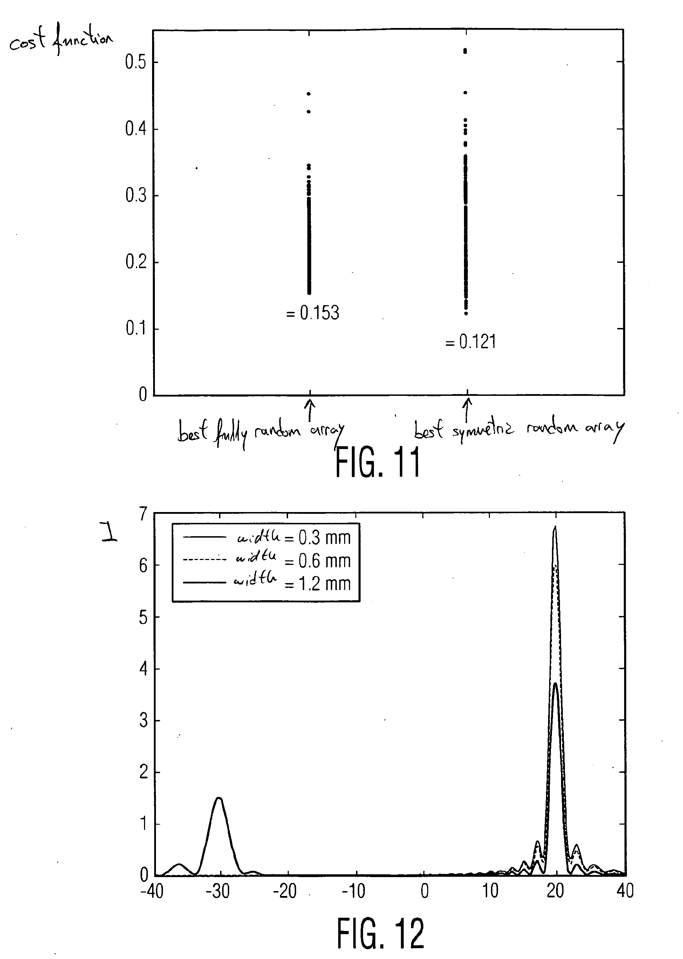

Figure 11 shows data obtained from a comparison of the cost functions of the fully random array and the symmetric random array (90 degree symmetry). Each dot is the cost function value obtained for one trial (lower value indicates a better performance). The data obtained indicates that the best symmetric random array yielded a better performance than the best fully random array.
Figure 12 shows effect on field intensity in arbitrary units on the ordinate, of element width on off-axis focusing for three one-dimensional linear arrays (non-random) with 36 mm aperture centered at x=0. The aperture size (product of the element width and the number of elements) was kept the same for all three cases. The wavelength was 1.5 mm, and no grating lobes were observed for the first two cases (widths = 0.3 and 0.6 mm). A grating lobe was observed at the width of 1.2 mm. Even when no grating lobes are present, smaller element widths were observed to lead to better signal intensity (0.3 mm vs. 0.6 mm). Without being limited by any particular theory or mechanism of action, this correlation is because the directivity of a smaller element is better than for a large element. When the width is increased to 1.2 mm, the signal intensity at the focus further drops due to increasing size and the appearance of the grating lobe. Thus, these data indicate that an element width that is smaller rather than larger yields superior results.

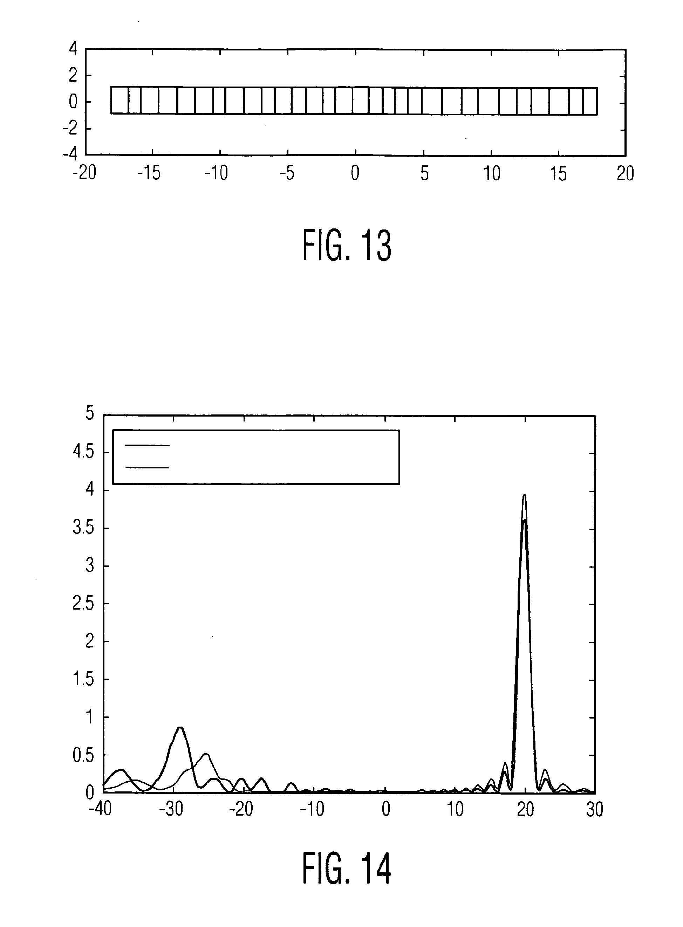

Figure 13 shows a one-dimensional random linear array The panel shows a random array with element sizes within the range between 0.9 mm to 1.5 mm.
Figure 14 shows the effect on field intensity of re-distributing the random elements in the one-dimensional random arrays shown in Figure 13. Better steering to the right (positive x) and reduced grating lobes are seen for the sorted array, especially when the beam is focused at x=20 mm on the plane (z = 30 mm).

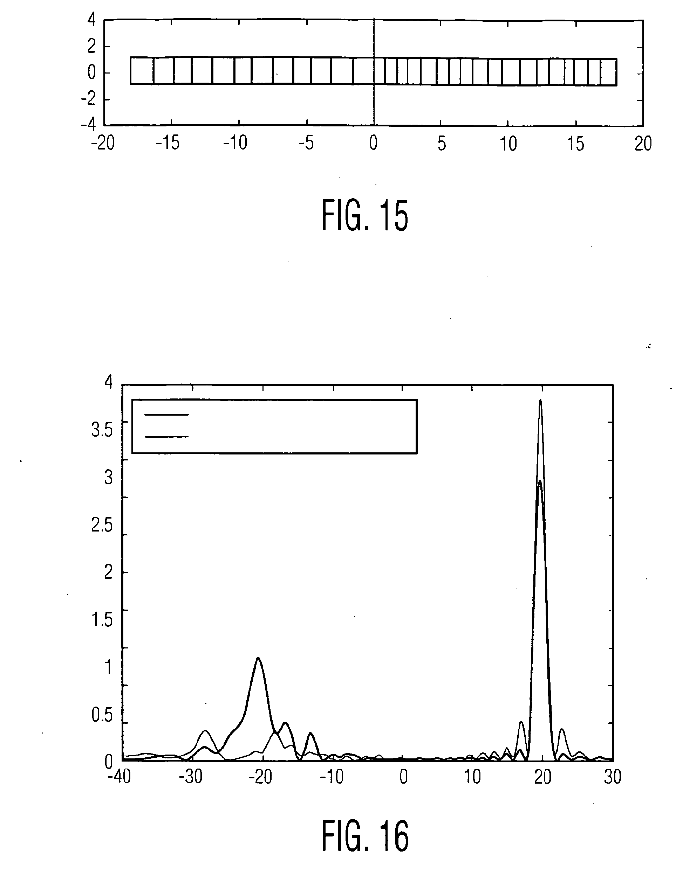

Figure 15 shows random arrays having two different distributions for element sizes:
   one distribution uniformly distributed over the interval 0.7 mm to 1.3 mm (18 elements), and the other 1.2 mm to 1.8 mm (12 elements). The total aperture size is the same as in Figure 13 (36 mm). Two configurations that are mirror images of each other are shown, one in which the larger elements are distributed over to the left (top panel), and the other in which the larger elements are distributed to the right (bottom panel).
Figure 16 shows the field intensity from the random array transducers having the two different distributions for element sizes that are shown in Figure 15. The function identified as "[Large; small]" shows data from the top panel shown in Figure 15 in which the larger elements are distributed to the left, and the function identified as "[Small; large]" shows data from the bottom panel in Figure 15 in which the larger elements are distributed to the right. The array was set to focus at 20 mm off axis at a distance of 30 mm from the array (i.e. x = 20, z = 30 mm). The data show that better steering (to the right) was achieved for the array indicated as [Small; large].
Figure 17 shows a two-dimensional random array (top) and a sorted version of this array (bottom). The sorting was done within each row to place the smaller elements to the left. (Small variations in element sizes are not apparent at the resolution of the plot.)
Figure 18 shows field intensity for the two-dimensional semi-random arrays as shown in Figure 17. For comparison, the beam plot from a non-random array is also shown. As in the previous result, the data observed here show that the sorted random array yielded superior performance.

Ultrasound elements can be made of lead zirconate titanate (PZT), polymer piezo-electric material or piezo-composite material.

A fully random one-dimensional array generally leads to asymmetric behavior when the beam is fired to one or the other side of the array - for example, the grating lobes might be small when steered to the left, but large when steered to the right. This phenomenon occurs when the mean of the sizes of the elements is larger on the left side compared to that on the right side. A similar situation exists in the two-dimensional array case.

In order to describe this invention, a convention is adopted that an array of ultrasound elements lies in the x-y plane at z=0, with its axis aligned with the z-axis. The array is fired to focus at different points, all lying in the same x-y plane at a certain distance from the array, e.g., the different points lying on a circle in the x-y plane. Essentially, the array is fired to focus in different directions. ["on a circle" is appropriate only for 2D, not 1D arrays]

Comparing the behavior of the array for the different directions, the grating lobe level would be small for certain directions, but not for others. Hence, unless a very large number of cases are tried in an exhaustive search, use of the fully random array tends to produce results such that the grating lobes are low when steered to one direction, but large when steered to other directions.

The wide range of therapeutic ultrasound applications poses the need for arrays with various properties. For example, while "forward steering" is desired in an endorectal tranducer for the treatment of prostate cancer, backward steering is not important because the transducer can be withdrawn slightly to reach other areas. Even in situations where preferential steering is not of great importance, the design cost of the array is an ever present concern. Accordingly, an object of this invention is to provide an array in which array design is simpler than for currently available models, and the array does not suffer from asymmetric behavior during steering from one direction to another. Another object of this invention is to provide an array that exhibits improved focusing and therapy when the beam is steered to one side of the array.

In one embodiment of this invention, an improvement compared to the random array is obtained by enforcing a symmetry constraint. For example, in the one-dimensional array, instead of designing the entire aperture with 128 randomly sized/distributed elements, about half of the aperture is designed using 64 elements and the other half is taken as the mirror image of the first half. In the case of two-dimensional arrays, only an angular (sector) portion of the array is designed using random elements, and the rest of the sectors in the array are filled with same elements by rotating the locations of all the elements in the first sector by appropriate angles. The present invention also includes a transducer that has a curved surface arranged in three dimensions with depth.

In this application, the phrase "fully random array" refers to arrays that have no symmetry constraints. Phrases "semi-random array" and "symmetric random array" refer to one-dimensional and two-dimensional random arrays respectively that have at least one degree of symmetry.

It is advantageous that when a symmetric constraint is enforced, the design of the array is made simpler and the array does not suffer from asymmetric behavior from one direction to another. For a one-dimensional case, only half the number of elements needs to be optimally selected, and the other half is selected by symmetry. For a two-dimensional case, when symmetry along x and y-axes are desired, only one fourth of the array needs to be designed and the other three quarters are selected by rotational symmetry. This greatly simplifies the design of an optimal random array.

In some embodiments, the array has rotational symmetry. For example, the array is partitioned into several portions, for instance, identical portions. For example, a circular geometry is split into several sectors, each sector containing aperiodic and/or random elements; however, all the sectors are transposable, for instance rotating one sector about the center of the array produces the appearance of another sector. An exemplary embodiment includes a circular two-dimensional array with one-quarter symmetry. In other embodiments, a 1/N symmetry is produced, in which N represents an integer greater than or equal to two.

In addition to design advantages, there are other advantages to a symmetric random array at the calibration and testing stage. For a random array, impedance matching needs to be done for each element. Such a procedure is expensive and time-consuming especially when repeated for each possible frequency of operation. When symmetry is enforced, only the designed portion of the array needs to be tested. For example, for a two-dimensional array where only one-fourth of the array is designed, only one-fourth of the array needs to be tested and calibrated. Also, if *a priori* look-up tables are used for storing excitation parameters for different focal regions, then only one-fourth of the focal regions needs to be calibrated.

One concern that might arise in reducing the number of designed elements through symmetry is that the richness in randomness is reduced. However, this is not the case as the following consideration and results obtained herein show. For a two-dimensional array, assuming circularly shaped elements, there are three unknowns: the x and y coordinates of the center of the elements, and the radius of the element. Even by quantizing these three quantities to five levels, the number of possible combination for an exhaustive search of 128 elements would be 10⁸⁹ (ignoring the non-overlapping constraint for simplicity). However, with only 32 elements to be designed (one-fourth of 128), the number of possible combinations is 10²², still sufficiently rich. Introduction into arrays of symmetry leads to adequate designs and, as shown by data observed in the examples herein, is found to yield arrays that perform better than fully random arrays. Moreover, there is some evidence that the best fully random array would tend to converge to some degree of symmetric array.

In systems provided herein, an important feature is a symmetry constraint in the random array. For example, in the one-dimensional case, instead of designing a one-dimensional array with 128 elements randomly filling the full aperture, only about half the aperture with 64 elements is designed. A mirror image of the elements is used to fill the other half. In the two-dimensional case with circular aperture, only a sector portion (e.g. 90 degree angle) of the array is designed using random arrays. The other sectors are filled using the same set of elements by rotating their locations through appropriate angles (90, 180 and 270 degrees) about the center of the array.

The bottom panel of Figure 1 shows such an embodiment of a one-dimensional semi-random array. In order to evaluate and compare this semi-random array with a fully random array, simulations in FIELD II were done. (Field II is a set of programs for simulating ultrasound transducer fields and ultrasound imaging using linear acoustics.) One-dimensional random arrays were simulated with an aperture size of 48 mm. The total number of elements for the two cases was 32. The element sizes were randomly distributed to be from 1.25 mm to 1.75 mm, having a mean size of 1.5 mm (no kerf was used in the simulations for simplicity). The frequency of operation was 1.5 MHz. For the fully random case, the entire aperture of 48 mm was filled with 32 elements. For the symmetric (scmi-random) array case, half the array size of 24 mm was filled with 16 elements and then mirrored to obtain the other half.

Figure 2 shows the beam pattern from one realization of the fully random one-dimensional array for two foci that were on either side of the array at a plane 80 mm from the array. The grating lobe when the focus was at +20 mm was 28% of the main lobe intensity. Performance was observed to be superior when the beam was focused to the other side with the grating lobe being only 12% of the main lobe. Since in general, there is no preferential one-sided steering, the cost function for this realization is 28%.

Figure 3 shows the beam pattern observed from one realization of the semi-random array. The beam pattern is the same whether the focus is at +20 or -20 mm, with a cost function of 21%. It should be pointed out that a direct comparison of results in Figures 2 and 3 is not possible, since these are for one particular trial of the random arrays. In prior art, 250 trials have been done for the optimal array selection. E.B. Hutchinson, et al., Design & optimization of an aperiodic ultrasound phased array for intracavity prostate thermal therapies, 23(5) MED. PHYS. 767-76 (1996). Accordingly, Figure 4 shows the cost functions for 250 trials, plotted as one dot for each realization. The best symmetric (semi-random) array was observed to have a better performance than the best fully random array.

In order to further compare the fully random and semi-random one-dimensional array, a total of 10,000 trials were conducted. Figure 5 shows the field intensity from the best (optimal) fully random array. The grating lobe intensity was observed to be much reduced compared to that in Figure 2. The beam patterns when the foci were at +20 and -20 mm appeared to be somewhat symmetric. Figure 6 shows the field intensity for the optimal semi-random array. The beam pattern (field intensity) for the two foci were observed to be symmetric and to show even smaller grating lobes compared to the optimal fully random array. Figure 7 shows the cost function plots for the two arrays for 10,000 trials, which also corroborate the better behavior of the symmetric (semi-random) array in comparison to the fully random array.

Further comparison of the optimal fully and semi-random (symmetric) arrays (selected after 10,000 trials) was performed to determine the effect of changing the focal locations to more than two points. Figure 8 shows the beam plots obtained for varying the range of the focus from -30 mm to 30 mm. It can be seen that the optimal semi-random array yielded a lower cost function compared to the optimal fully random array. The fully random array produced unacceptably high grating lobes for focusing at -30 mm and 30 mm, comparable to the non-random array with a constant width of 1.5 mm.

Another embodiment for two-dimensional random arrays is now described. The array contains a 40 mm circular aperture filled with 120 elements. The elements in this embodiment are circular in shape with random radii uniformly distributed between 1 and 2 mm. The frequency of operation was 1.5 MHz. Figure 9 shows one realization of the fully random two-dimensional array. Figure 10 shows one realization of the two-dimensional symmetric random array with a 90-degree symmetry. For this array, 30 elements were randomly chosen to fit one quadrant, and then the other quadrants were filled by placing the same elements by rotating the quadrant by 90, 180, and 270 degrees.

To compare the cost functions for the fully random and symmetric random arrays, beam plot simulations were done in FIELD II for 8 focal locations (one by one; not simultaneous multiple foci). The 8 focal spots were located on a circle and lying in a plane 80 mm from the array. These 8 focal points were separated by 45 degrees apart on the circle that had a radius of 20 mm. The worst ratio between grating lobe to main lobe is reported. Because of computational time constraints, 250 trials were conducted rather than 10,000 for the one-dimensional case. However, it is believed that statistical significance was achieved.

Figure 1 shows the cost function observed for these 250 trials for each of the fully random and the symmetric two-dimensional random arrays. It was observed that with even these many trials, the symmetric random array yielded a better performance. Hence, even though the amount of randomness is reduced for the symmetric array, the performance is actually better than the one with fully randomness.

In accordance with the principles of the present invention, another embodiment is a two-dimensional random array aperture that is non-circular in shape, e.g., rectangular, triangular, pentagonal, n-gonal, elliptical, trapezoidal, rhomboidal, or square.

In other embodiments, the sector angle for symmetry is not limited to 90 degrees, and could be, for example, in the range 0 to 180 degrees. Angles between 20 and 180 degrees enable a richer pattern of ultrasound elements for symmetric reproduction.

The above embodiments can be used in any application where focused ultrasound is used to thermally ablate tissue. Typical examples include therapy for brain lesions, uterine fibroids, liver tumor ablation, breast tumor ablation and the like. The embodiments further include methods of use of ultrasound for clot dissolution, for example, for stroke or deep venous thrombosis (DVT) patients.

The above embodiments can also be used in applications where thermally induced bioeffects are needed, such as localized gene therapy, drug delivery, and protein delivery.

While the random arrays are intended for therapy purposes, it is conceivable that the above embodiments can be applied to ultrasound imaging arrays as well.

In another embodiment of the invention, the elements are arranged such that the larger elements are closer to the preferred direction of steering and smaller elements are located on the other (distal) side of array, yielding better focusing and therapy when the beam is steered to one side of the array.

Smaller elements are advantageous compared to larger elements in several respects: they have a relatively more isotropic beam pattern compared to large elements, producing ultrasound beams that are easier to steer the from smaller elements off axis, especially close to the array. Further, the grating lobes are lower when smaller elements are used. The reduction of grating lobes leads to improved energy deposition in the main lobe, a feature that is especially useful when the beam is steered off-axis.

An example of a simple non-random array illustrates the above embodiment. Three one-dimensional linear arrays with constant element sizes (non-random array) were simulated using the FIELD II program. The total aperture size was the same for all three cases, 36 mm. The element widths for the three cases were 0.3 mm, 0.6 mm, and 1.2 mm, respectively, and the numbers of elements were 120, 60, and 30 respectively. The frequency was set to 1.0 MHz, corresponding to a wavelength of 1.5 mm. For simplicity, no kerf was used in the simulations, and therefore the spacing between the elements is the determined by the widths of the elements. No grating lobes were observed for the first two cases.

Figure 12 shows the field intensity observed from the three arrays when the beam was set to focus 20 mm off-axis at a plane 30 mm from the aperture. The data show that the signal intensity at the focus decreases progressively with increasing element size. Even when no grating lobes are present, smaller element widths lead to better signal intensity (0.3 mm vs. 0.6 mm). This is because the directivity of smaller elements is better. When the width is increased to 1.2 mm, the signal intensity at the focus further drops due to increasing size and the appearance of the grating lobe. Thus, it is preferential to have smaller elements.

Random arrays previously described minimize grating lobes even when the element spacing is larger than λ/2, where λ is the acoustic wavelength (E. Hutchinson et al., Aperiodic ultrasound phased array, PCT patent application serial number WO 97/17018; J.F. Hand et al., Arrays of quasi-randomly distributed ultrasound transducers, PCT patent application serial number WO 00/49598). However, they tend to have poorer steering capability when the beam is steered off-axis, which is here considered to be correlated with to the use of larger elements. To illustrate this, another example is provided. Figure 13 shows two random array configurations with the same set of randomly sized elements. The element sizes were chosen from a uniform distribution between 0.9 mm to 1.5 mm (quantized to the nearest 0.05 mm). The number of elements was 30 and the total aperture size was the same as in the previous example (36 mm). The array in the bottom panel of Figure 13 used the same set of elements in the top panel, and the elements in the bottom panel further were sorted to place the elements in an ascending order of sizes from left to right. The mean element size for both the arrays was 1.2 mm.

Figure 14 shows the beam patterns from the two arrays when the focusing was set to 20 mm to the right at a plane 30 mm from the aperture. Both random arrays show an improvement compared to the array with a fixed element size of 1.2 mm (black curve in Figure 12 - if you really need color plots, please let us know; otherwise the different plots would look similar when printed in grayscale). However, the sorted random array shows an even greater improvement, compared to the unsorted array that used the same set of elements. The improvement was facilitated by locating the larger elements that have relatively poorer element directivity closer to the focal spot. The smaller elements that have better directivity were placed farther from the focal spot. The sorted random array was observed to have a reduced grating lobe. Such a sorted array thus was found to have a superior steering capability to one side of the array. The steering on the other side of the array, while relatively poorer due to the smaller elements, is capable for internal transducers such as an endorectal probe to be compensated, i.e., it is usually possible to be able to steer successfully into the body, and focusing to the other side can subsequently easily be accomplished by simply retracting the transducer slightly.

An embodiment provides use of an arrangement of random elements in which larger elements are located closer to the preferred direction of steering, and smaller elements are located to the other side, further from the preferred direction. As shown in Figure 14, the focusing gain is improved and the grating lobes are reduced for this array compared to those observed for a random array having however the same set of element sizes.

Optimization has been performed in related embodiments in order to choose the best distribution of elements as disclosed in patents. E. Hutchinson et al., Aperiodic ultrasound phased array, PCT patent application serial number WO 97/17018; J.F. Hand et al., Arrays of quasi-randomly distributed ultrasound transducers, PCT patent application serial number WO 00/49598. However, it should be noted that the best configuration as described in those patents lacks the preferred steering capability as provided by the methods herein. Accordingly, they would perform more poorly compared to the proposed array when preferential steering is needed.

Figure 13, bottom panel shows an embodiment of a preferentially steered array, in which a one-dimensional random array is first chosen, and then the elements are sorted in ascending order of size. The beam patterns are determined through simulations or experiments for a set of focal spots in one direction of the array. An optimization scheme is implemented with different trials of sorted random arrays in order to select the best configuration, i.e., the array that has the best performance as determined by the focusing gain and/or grating lobe levels. Additionally, the optimization is made that includes good steering up to a specific angle in the backward direction.

Figure 15 shows another example of an embodiment of one-dimensional linear arrays. Here the array is made from two distributions of element sizes: one distribution has a larger mean size and the other has a smaller mean size. Preferably, the number of elements in both sets is selected such that each set fills half of the array. The larger distribution elements fill the side of the array that is used to obtain superior or preferential steering.

Figure 16 shows the data obtained from beam patterns resulting with the random arrays shown in Figure 15 in which focusing was done to the right side. It can be seen that with the arrangement in which the elements from the larger size distribution are placed in the right half of the array, it is observed that steering is significantly improved, compared to the arrangement having the smaller elements placed in the right half. Optimization strategies can further be employed to choose among distributions that have the best performance in terms of focusing gain and or grating lobe levels.

It should be readily apparent to those skilled in the art that if backward steering is preferentially needed, then the above embodiments can be employed by switching the locations of the larger and smaller elements.

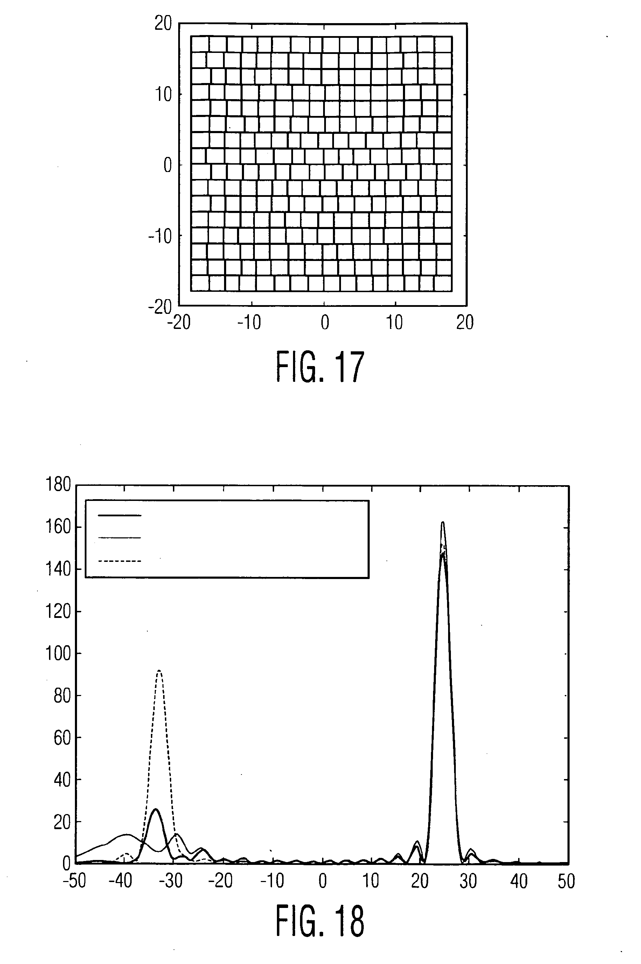

An additional embodiment is a two-dimensional array that requires improved or superior steering in one direction, for example, where the target tissue is located to one side of the acoustic window, e.g. sub-costal access to the heart. To illustrate the two-dimensional application, a semi-random two-dimensional array with 256 elements (16x16) was simulated (Figure 17). The aperture size was 36 mm x 36 mm square, and the element sizes in the x direction were chosen from a uniform random distribution between 1.75 mm and 2.75 mm (quantized in 0.05 mm steps). For simplicity, the element sizes were of constant size equal to 2.25 mm in the y direction. Different random distributions were chosen for each of the 16 rows, and thus the two-dimensional array consisted of a set of one-dimensional random arrays. Since the kerf was set to zero, the element spacing was determined by the element widths. Figure 18 shows the beam pattern at 1 MHz (λ=1.5 mm) in which the focus was set to 25 mm to the right of the axis at a distance of 80 mm from the aperture, for both the original array and the sorted array. Also shown for comparison is the beam plot from a non-random array with constant element sizes of 2.25 mm. These data show, as in the embodiments above, that the sorted random array yields superior results.

The above embodiment can also be extended to one-dimensional or two-dimensional arrays in which the elements are located on a curved surface, e.g., a spherical shell.

The above embodiment can be used in any application for which focused ultrasound is used to thermally ablate tissue and the transducer needs to be advanced into the body. Typical applications are in intra-cavity transducers such as are used for prostate treatment, catheter based transducers that may not be able to advance beyond an anatomical site due to blockage or danger from disrupting a clot, laparoscopic therapy transducers, and transducers for uterine fibroid treatment. Catheter based applications include clot dissolution and electrophysiology (EP) applications for correcting the abnormal electrical firing of the heart cells.

Applications that are trans-thoracic or trans-abdominal are also within the scope of the embodiments herein, for example, applications in which the therapeutic volume is located consistently asymmetrically shifted from the acoustic window. These applications include access to the heart from a sub-costal or specific rib spacing, or trans-abdominal access to the liver or other organs to avoid the intestines or bladder. The invention can also be used in applications for which thermally induced bioeffects such as localized gene therapy, drug delivery, protein delivery, etc. are needed.

In general, randomly selected element sizes and locations are produced by any standard means of generating random numbers of values. These means include random number generators, random number tables, random distributions and quasi random distributions.

The above embodiments are generally applicable to varieties and sizes of ultrasound elements. This includes, but is not limited to, elements with sizes ranging from about 0.1 mm to about 100 mm range.

It will furthermore be apparent that other and further forms of the invention, and embodiments other than the specific and exemplary embodiments described above, may be devised without departing from the spirit and scope of the appended claims and their equivalents, and therefore it is intended that the scope of this invention encompasses these equivalents and that the description and claims are intended to be exemplary and should not be construed as further limiting.

## Claims

1. A phased ultrasound array for an ultrasound generating device comprising a symmetrically constrained plurality of randomly selected ultrasound elements partitioned into N portions with 1/N symmetry, in which N represents an integer greater than or equal to two, wherein the randomly selected ultrasound elements of each portion of the array are arranged symmetrically about one of (i) a point or (ii) a dividing line in the array with respect to element sizes, shapes, and element locations, wherein the randomly selected ultrasound elements of a portion include at least one element of the plurality of elements which has a size and/or shape that is different from the remainder of the plurality of elements in the respective portion and/or at least one inter-element spacing that is different from a remainder of inter-element spacings in the respective portion.

2. The phased ultrasound array according to claim 1, wherein sizes and/or shapes of the ultrasound elements are randomly selected.

3. The phased ultrasound array according to claim 1, wherein locations of the ultrasound elements are randomly selected.

4. The phased ultrasound array according to claim 1, wherein a plurality of the distances between the locations of centers of mass of adjacent ultrasound elements is aperiodic.

5. The phased ultrasound array according to claim 1, wherein the phased ultrasound array comprises a preferentially steered phased ultrasound array comprising a plurality of ultrasound elements, wherein a majority of larger elements of the ultrasound array are arranged on a first portion of the array and a majority of smaller ultrasound elements are arranged on a second portion of the array, wherein the first portion is closer to a preferred direction of steering than the second portion.

6. The phased ultrasound array according to claim 5, wherein a plurality of ultrasound elements within each portion are randomly arranged.

7. The phased ultrasound array according to claim 5, wherein the sizes of the ultrasound elements are randomly selected from two distributions of element sizes: a first distribution of ultrasound element sizes, and a second distribution of ultrasound elements sizes, wherein mean size in the first distribution is greater than in the second distribution.

8. The phased ultrasound array according to claim 7, wherein the elements of the first portion are randomly selected from the first distribution and the elements of the second portion are randomly selected from the second distribution.

9. The phased ultrasound array according to claim 8, wherein the first portion and the second portion each constitute half of the area of the array.

10. The phased ultrasound array according to claim 1, wherein the array is one-dimensional or two-dimensional or three-dimensional.

11. The phased ultrasound array according to claim 1, wherein the ultrasound elements further comprise a material selected from the group consisting of: lead zirconate titanate (PZT), polymer piezo-electric material, and piezo-composite material.

12. The phased ultrasound array according to claim 1, wherein the ultrasound elements are arranged on a curved surface.

13. A method of making a phased ultrasound array, the method comprising:
selecting randomly sized ultrasound elements; and,
arranging the randomly sized ultrasound elements symmetrically on the array, wherein arranging symmetrically includes partitioning the array into N portions of the randomly sized ultrasound elements with 1/N symmetry, in which N represents an integer greater than or equal to two.

14. The method of making a phased ultrasound array according to claim 13, wherein larger ultrasound elements are arranged on a first portion of the array and smaller ultrasound elements are arranged on a second portion of the array, wherein the first portion is closer to a preferred direction of steering than the second portion.

15. The method of making a phased ultrasound array according to claim 14, further comprising optimizing the arrangement of ultrasound elements for steering in an additional direction, wherein optimizing is maintaining focusing gain and minimizing grating lobes for both the preferred direction of steering and the additional direction.

## Patentansprüche

1. Ultraschall-Phased-Array für eine Ultraschall erzeugende Vorrichtung, mit einer symmetrisch begrenzten Vielzahl von zufällig ausgewählten Ultraschallelementen, die in N Abschnitte mit 1/N-Symmetrie partitioniert sind, wobei N eine Ganzzahl gleich oder größer als zwei ist, wobei die zufällig ausgewählten Ultraschallelemente für jeden Abschnitt des Arrays symmetrisch um entweder (i) einen Punkt oder (ii) eine Teilungslinie in dem Array in Bezug auf Elementgrößen, Formen und Elementpositionen angeordnet sind, wobei die zufällig ausgewählten Ultraschallelemente eines Abschnitts mindestens ein Element der Vielzahl von Elementen umfassen, welches eine Größe und/oder Form hat, die sich von dem Rest der Vielzahl von Elementen in dem betreffenden Abschnitt unterscheidet und/oder mindestens einen Zwischenelementabstand, der sich von einem Rest der Zwischenelementabstände in dem betreffenden Abschnitt unterscheidet.

2. Ultraschall-Phased-Array nach Anspruch 1, wobei Größen und/oder Formen der Ultraschallelemente zufällig ausgewählt sind.

3. Ultraschall-Phased-Array nach Anspruch 1, wobei Positionen der Ultraschallelemente zufällig ausgewählt sind.

4. Ultraschall-Phased-Array nach Anspruch 1, wobei eine Vielzahl der Abstände zwischen den Positionen der Massenmitte von benachbarten Ultraschallelementen aperiodisch ist.

5. Ultraschall-Phased-Array nach Anspruch 1, wobei das Ultraschall-Phased-Array ein vorzugsweise gesteuertes Ultraschall-Phased-Array mit einer Vielzahl von Ultraschallelementen umfasst, wobei eine Mehrzahl von größeren Elementen des Ultraschall-Arrays auf einem ersten Abschnitt des Arrays angeordnet ist und eine Mehrzahl von kleineren Ultraschallelementen auf einem zweiten Abschnitt des Arrays angeordnet ist, wobei der erste Abschnitt einer bevorzugten Steuerungsrichtung näher ist als der zweite Abschnitt.

6. Ultraschall-Phased-Array nach Anspruch 5, wobei eine Vielzahl von Ultraschallelementen innerhalb jedes Abschnitts zufällig angeordnet ist.

7. Ultraschall-Phased-Array nach Anspruch 5, wobei die Größen der Ultraschallelemente zufällig aus zwei Verteilungen von Elementgrößen ausgewählt sind:
einer ersten Verteilung von Ultraschallelementgrößen und einer zweiten Verteilung von Ultraschallelementgrößen, wobei die mittlere Größe in der ersten Verteilung größer ist als in der zweiten Verteilung.

8. Ultraschall-Phased-Array nach Anspruch 7, wobei die Elemente des ersten Abschnitts zufällig aus der ersten Verteilung ausgewählt werden und die Elemente des zweiten Abschnitts zufällig aus der zweiten Verteilung ausgewählt werden.

9. Ultraschall-Phased-Array nach Anspruch 8, wobei der erste Abschnitt und der zweite Abschnitt jeweils die Hälfte der Fläche des Arrays bilden.

10. Ultraschall-Phased-Array nach Anspruch 1, wobei das Array eindimensional oder zweidimensional oder dreidimensional ist.

11. Ultraschall-Phased-Array nach Anspruch 1, wobei die Ultraschallelemente weiterhin ein Material ausgewählt aus der Gruppe bestehend aus Bleizirkonattitanat (PZT), polymeres piezoelektrisches Material und Piezo-Verbundmaterial umfassen.

12. Ultraschall-Phased-Array nach Anspruch 1, wobei die Ultraschallelemente auf einer gekrümmten Oberfläche angeordnet sind.

13. Verfahren zur Herstellung eines Ultraschall-Phased-Arrays, wobei das Verfahren Folgendes umfasst:
Auswählen von zufällig bemessenen Ultraschallelementen; und
Anordnen der zufällig bemessenen Ultraschallelemente symmetrisch auf dem Array, wobei das symmetrische Anordnen das Partitionieren des Arrays in N Abschnitte von zufällig bemessenen Ultraschallelementen mit 1/N-Symmetrie umfasst, wobei N eine Ganzzahl größer oder gleich zwei darstellt.

14. Verfahren zur Herstellung eines Ultraschall-Phased-Arrays nach Anspruch 13, wobei größere Ultraschallelemente auf einem ersten Abschnitt des Arrays angeordnet sind und kleinere Ultraschallelemente auf einem zweiten Abschnitt des Arrays angeordnet sind, wobei der erste Abschnitt einer bevorzugten Steuerungsrichtung näher ist als der zweite Abschnitt.

15. Verfahren zur Herstellung eines Ultraschall-Phased-Arrays nach Anspruch 14, das weiterhin das Optimieren der Anordnung von Ultraschallelementen zum Steuern in eine zusätzliche Richtung umfasst, wobei das Optimieren das Aufrechterhalten der Fokussierungsverstärkung und das Minimieren von Rasterkeulen für sowohl die bevorzugte Steuerungsrichtung als auch die zusätzliche Richtung umfasst.

## Revendications

1. Ensemble ultrasonore en phase pour un dispositif de génération ultrasonore comprenant une pluralité d'éléments ultrasonores, sélectionnés de manière aléatoire, agencés symétriquement, et partitionnés en N parties avec une symétrie 1/N, dans lequel N représente un nombre entier supérieur ou égal à deux, dans lequel les éléments ultrasonores sélectionnés de manière aléatoire de chaque partie de l'ensemble sont agencés symétriquement autour de l'un de (i) un point ou (ii) une ligne de division dans l'ensemble par rapport à des tailles d'éléments, des formes et des emplacements d'éléments, dans lequel les éléments ultrasonores sélectionnés de manière aléatoire d'une partie comprennent au moins un élément de la pluralité d'éléments qui a une taille et/ou une forme différentes de celles du reste de la pluralité d'éléments dans la partie respective et/ou au moins un espacement entre éléments qui est différent des autres espacements entre éléments dans la partie respective.

2. Ensemble ultrasonore en phase selon la revendication 1, dans lequel les tailles et/ou les formes des éléments ultrasonores sont sélectionnées de manière aléatoire.

3. Ensemble ultrasonore en phase selon la revendication 1, dans lequel les emplacements des éléments ultrasonores sont sélectionnés de manière aléatoire.

4. Ensemble ultrasonore en phase selon la revendication 1, dans lequel une pluralité de distances entre les emplacements des centres de masse des éléments ultrasonores adjacents est apériodique.

5. Ensemble ultrasonore en phase selon la revendication 1, dans lequel l'ensemble ultrasonore en phase comprend un ensemble ultrasonore en phase orienté de manière préférentielle comprenant une pluralité d'éléments ultrasonores, dans lequel une majorité d'éléments plus grands de l'ensemble ultrasonore sont agencés sur une première partie de l'ensemble et une majorité d'éléments ultrasonores plus petits sont agencés sur une seconde partie de l'ensemble, dans lequel la première partie est plus proche d'une direction préférée d'orientation que la seconde partie.

6. Ensemble ultrasonore en phase selon la revendication 5, dans lequel une pluralité d'éléments ultrasonores à l'intérieur de chaque partie sont agencés de manière aléatoire.

7. Ensemble ultrasonore en phase selon la revendication 5, dans lequel les tailles des éléments ultrasonores sont sélectionnées de manière aléatoire parmi deux distributions de tailles d'éléments : une première distribution de tailles d'éléments ultrasonores, et une seconde distribution de tailles d'éléments ultrasonores, dans lequel la taille moyenne dans la première distribution est supérieure à celle dans la seconde distribution.

8. Ensemble ultrasonore en phase selon la revendication 7, dans lequel les éléments de la première partie sont sélectionnés de manière aléatoire parmi la première distribution et les éléments de la seconde partie sont sélectionnés de manière aléatoire parmi la seconde distribution.

9. Ensemble ultrasonore en phase selon la revendication 8, dans lequel la première partie et la seconde partie constituent chacune la moitié de la surface de l'ensemble.

10. Ensemble ultrasonore en phase selon la revendication 1, dans lequel l'ensemble est unidimensionnel, bidimensionnel ou tridimensionnel.

11. Ensemble ultrasonore en phase selon la revendication 1, dans lequel les éléments ultrasonores comprennent en outre un matériau sélectionné dans le groupe se composant de : plomb zirconate titanate (PZT), matériau piézo-électrique polymérique, et matériau piézo-composite.

12. Ensemble ultrasonore en phase selon la revendication 1, dans lequel les éléments ultrasonores sont agencés sur une surface incurvée.

13. Procédé consistant à réaliser un ensemble ultrasonore en phase, le procédé comprenant :
la sélection d'éléments ultrasonores calibrés de manière aléatoire ; et
l'agencement symétrique des éléments ultrasonores calibrés de manière aléatoire sur l'ensemble, dans lequel l'agencement symétrique comprend le partitionnement du réseau en N parties des éléments ultrasonores calibrés de manière aléatoire avec une symétrie 1/N, dans lequel N représente un nombre entier supérieur ou égal à deux.

14. Procédé consistant à réaliser un ensemble ultrasonore en phase selon la revendication 13, dans lequel les éléments ultrasonores plus grands sont agencés sur une première partie de l'ensemble et les éléments ultrasonores plus petits sont agencés sur une seconde partie de l'ensemble, dans lequel la première partie est plus proche d'une direction préférée d'orientation que la seconde partie.

15. Procédé consistant à réaliser un ensemble ultrasonore en phase selon la revendication 14, comprenant en outre l'optimisation de l'agencement d'éléments ultrasonores pour l'orientation dans une autre direction, dans lequel l'optimisation consiste à maintenir le gain de focalisation et à minimiser les lobes secondaires pour la direction préférée d'orientation et pour l'autre direction.
